# EUROPEAN PATENT APPLICATION

(11) **EP 4 506 356 A1**
(43) Date of publication of application: **12.02.2025**
(21) Application number: 22934368.6
(22) Date of filing: 02.04.2022
(51) Int. Cl.: C07K 5/062, C12N 1/21

(54) **METHANOL-INDUCIBLE PROMOTER AND USE THEREOF IN PREPARATION OF L-ALANYL-L-GLUTAMINE**

(71) Applicant: Wuhan Grand Hoyo Co., Ltd., Wuhan, Hubei 430074 (CN)
(72) Inventor: LIN, Lichun, Wuhan, Hubei 430074 (CN); PI, Li, Wuhan, Hubei 430074 (CN); SUN, Yue, Wuhan, Hubei 430074 (CN); XING, Panpan, Wuhan, Hubei 430074 (CN); SU, Haixia, Wuhan, Hubei 430074 (CN); ZHA, Liyan, Wuhan, Hubei 430074 (CN); HE, Jiajun, Wuhan, Hubei 430074 (CN); ZHU, Yuhan, Wuhan, Hubei 430074 (CN); CHEN, Lei, Wuhan, Hubei 430074 (CN); LI, Xiaobo, Wuhan, Hubei 430074 (CN); YE, Miao, Wuhan, Hubei 430074 (CN); WEI, Zixiang, Wuhan, Hubei 430074 (CN)
(74) Representative: Epping - Hermann - Fischer
(86) International application number: PCT/CN2022/085076
(87) International publication number: WO 2023/184539

(57) **Abstract**

Provided are a methanol-inducible promoter and the use thereof in the preparation of L-alanyl-L-glutamine. A nucleotide sequence encoding the methanol-inducible promoter is as shown in SEQ ID NO: 6. Further disclosed is a fusion gene, which comprises the methanol-inducible promoter and amino acid ester acyltransferase, wherein the nucleotide sequence of the amino acid ester acyl-transferase is as shown in SEQ ID NO: 25.

## Description

### TECHNICAL FIELD

The present disclosure belongs to the field of genetic engineering/biosynthesis, and in particular relates to a methanol-inducible promoter and use thereof in the preparation of alanyl-glutamine dipeptide.

### BACKGROUND

Alanyl-glutamine dipeptide, L-alanyl-L-glutamine, has attracted extensive attention at home and abroad due to its advantages of high thermal stability and water solubility (586 g/L) and can be rapidly decomposed into L-glutamine in vivo, etc. This small peptide can make up for the shortage of glutamine and expand its clinical application range as an intravenous nutritional preparation.

At present, the methods for preparing alanyl-glutamine dipeptide are mainly divided into the following two categories: the first category is chemical synthesis methods, such as N-carbonyl-alanine anhydride method, protecting group method and D-2-halo-propionyl-L-glutamine ammonolysis method, etc. The above methods have obvious disadvantages, complicated reaction steps, many by-products, high reagent toxicity, and environmental pollution; the second category is microbial enzymatic conversion, the *ywfE* gene in *Bacillus subtilis* 168 encodes an L-amino acid ligase that can catalyze the synthesis of alanyl-glutamine dipeptide from the substrates L-alanine and L-glutamine, but the reaction requires the supply of ATP and the yield of the product is low. In addition, α-amino acid ester acyltransferase can be synthesized by *Empedobacter brevis* and *Sphingobacterium siyangensis,* which can efficiently synthesize alanyl-glutamine dipeptide using L-alanine methyl ester hydrochloride (L-Ala-OMe·HCl) and L-glutamine as substrates. The α-amino acid acyltransferase method has the advantages of simple reaction steps, green environmental protection, and no need for ATP, etc. However, the existing acyltransferase recombinant expression strains still have the disadvantage of low enzyme activity.

*Pichia pastoris* is one of the commonly used expression hosts for industrial enzyme production, belonging to a Generally Recognized as Safe (GRAS) microorganism certified by the FDA in the United States, and the secretion level of foreign proteins can reach g/L level, and high-density fermentation is easy to carry out. Promoter is an important factor in regulating protein expression. The strength of the promoter and the matching degree with the foreign proteins significantly affect the expression level of foreign proteins. The most widely used promoters in *Pichia pastoris* are P_{AOX1} (alcohol oxidase) and P_{GAP} (glyceraldehyde-3-phosphate dehydrogenase) promoters. P_{AOX1} promoter is strongly induced by methanol, but it is inhibited when there are glycerol, ethanol, and other carbon sources in the medium. P_{GAP} promoter is a strong constitutive promoter, and its expression level is closely related to the growth state of yeast. In addition to the two most commonly used promoters, methanol-inducible promoters such as the P_{FLD1} (formaldehyde dehydrogenase) promoter, the P_{DAS} (dihydroxyacetone synthase) promoter have been reported. Prielhofer et al., reported that the P_{G1} and P_{G6} promoters were induced by low concentrations of glucose and repressed by glycerol. In addition, researchers have also conducted a small amount of researches on some constitutive promoters such as P_{TEF1} (translation elongation factor 1) promoter, P_{YPT1} (GTP enzyme) promoter, and P_{GCW14} (GPI-anchored protein) promoter. However, the above uncommon promoters have not been widely used because they are more or less deficient in expression strength, induction conditions, strict regulation, etc. For example, P_{FLD1} requires not only methanol as the sole carbon source but also ammonium sulfate as a nitrogen source. The strength of P_{G1}, P_{G6}, and a few constitutive promoters is not sufficient to support large-scale expression of foreign proteins. Thus, the promoters available for *Pichia pastoris* are very limited, and it is essential to screen new promoters for the expression of different active proteins. An object of the present disclosure is to screen a methanol-inducible promoter, construct a *Pichia pastoris* recombinant expression vector, and secrete and express amino acid ester acyltransferase with high efficiency for the production of alanyl-glutamine dipeptide.

The U.S. Patent US8222386B2 discloses the use of three novel inducible promoters of *Pichia pastoris,* ADH1, GUT1, and ENO1, wherein ADH1 is induced by ethanol or glycerol. These novel promoters have not been compared with AOX1 and GAP and lack absolute expression values, thus their regulatory characteristics, expression strength, and applicability are unclear.

Reference 1: Hohenblum H, et al., (2004), Biotechnology and Bioengineering, 85:367-75, used vectors containing either the AOX1 promoter or the GAP promoter to secrete and express human trypsinogen 1, transformed Pichia pastoris, and screened the multi-copy recombinant strain. The results showed that for the expression of AOX1 promoter, and the expression amount of trypsinogen in the recombinant strain with more than 2 copies was reduced.

The amino acid ester acyltransferase AET derived from *Sphingobacterium siyangensis* SY1 was secreted and intracellularly expressed in *Pichia pastoris,* respectively. The intracellular and secreted expression were performed using vectors pPIC3.5K and pPIC9K containing the AOX1 promoter. The highest substrate conversion rate of KP_{3.5K}Ap1800 (intracellular) was as high as 80%, while KP9KAp1800 (secreted) was only 57% (Reference 2: Li Yimin, "Efficiently Synthesis Of L-Alanyl-L-glutamine by Whole-Cell Catalysis of Recombinant Microorganisms" [D], phD thesis, Dalian University of Technology, 2020). The promoter used therein is AOX1, which is a strictly methanol-inducible strong promoter, and the extracellular activity of the expressed amino acid ester acyltransferase is low.

### SUMMARY

In order to overcome the above-mentioned defects, the object of the present disclosure is to screen out a strong inducible promoter, construct a *Pichia pastoris* recombinant strain capable of efficiently secreting and expressing amino acid ester acyltransferase, and screen out a high-copy integrated recombinant strain with high amino acid ester acyltransferase activity and can be used for the preparation of alanyl-glutamine dipeptide.

In order to solve the above-mentioned problems, the present disclosure uses the transcriptome database of *Pichia pastoris* to search for a promoter of a class of target genes whose transcription level was significantly increased during the methanol induction period and constructs a series of inducible high-efficiency expression plasmids, screens out an optimal promoter by detecting the strength of the promoter using an amino acid ester acyltransferase (AET) from *Sphingobacterium* siyangensis, and then constructs a recombinant expression strain integrating multiple copies of the AET gene for use in the enzymatic conversion production of alanyl-glutamine dipeptide.

Specifically, in the present disclosure, based on the sequencing data of *Pichia pastoris* transcriptome, target genes whoes transcription level was significantly improved during the methanol induction period were screened, the promoter regions of the genes were analyzed using promoter prediction software, the promoter of the gene was amplified using PCR, the promoter and AET gene were assembled into a p9K plasmid with the original AOX1 promoter removed. The recombinant plasmid was linearized with BglII, then integrated into the *Pichia pastoris* genome through double exchange for expression. The recombinant strains were cultured and induced with BMGY and BMMY medium, respectively, and the AET enzyme activities expressed by the recombinant strains with different promoters were compared to screen out the promoter with the highest expression strength.

A first aspect of the present disclosure provides a promoter, a nucleotide sequence encoding the promoter is as shown in SEQ ID NO: 6.

A second aspect of the present disclosure provides a fusion gene including a promoter and an amino acid ester acyltransferase, wherein, the nucleotide sequence of the promoter is as shown in SEQ ID NO: 6 and the nucleotide sequence of the amino acid ester acyltransferase is as shown in SEQ ID NO: 25.

A third aspect of the present disclosure provides a recombinant vector including the promoter as described in the first aspect of the present disclosure or a fusion gene as described in the second aspect of the present disclosure.

In some specific embodiments, a backbone plasmid of the recombinant vector is pGAPZαA, pPICZαA, pPIC9, or p9K. More preferably, the backbone plasmid is p9K.

A fourth aspect of the present disclosure provides a *Pichia pastoris* engineered strain including the recombinant expression vector according to the third aspect of the present disclosure; and an expression host of the *Pichia pastoris* engineered strain is a wild-type host, a histidine-deficient host, or a protease-deficient host.

In some specific embodiments, the wild-type host is X33, the histidine-deficient host is KM71 or GS115, or the protease-deficient host is SMD1163, SMD1165, or SMD1168.

In some preferred embodiments, the expression host is *Pichia pastoris* strain GS115.

In some more preferred embodiments, the *Pichia pastoris* engineered strain is *Pichia pastoris* engineered strain G6 deposited with a deposit number of CCTCC NO: M 20211308.

A fifth aspect of the present disclosure provides a method for preparing an amino acid ester acyltransferase including the following steps:
culturing a seed solution including the *Pichia pastoris* engineered strain according to the fourth aspect of the present disclosure in a BMMY medium, and adding when the OD₆₀₀ value is 0.5 to 2, such as 1.0, an inducer, such as methanol, to induce expression of the amino acid ester acyltransferase; wherein a final concentration of the inducer ranges preferably from 0.5% to 1%.

In some specific embodiments, a duration of said induction expression ranges from 48 hours to 120 hours, such as 96 hours, or said induction expression lasts until an OD₆₀₀ value ranges from 20 to 30, to obtain a fermentation broth containing the amino acid ester acyltransferase.

Preferably, methanol is supplemented every 12 hours to 24 hours.

More preferably, the method further including: concentrating the fermentation broth to obtain a concentrated enzyme solution; preferably, said concentrating is ultrafiltration concentrating.

In some preferred embodiments, the seed solution is prepared by:
(a) inoculating the *Pichia pastoris* engineered strain into a medium such as BMGY medium;
(b) culturing at 25°C to 30°C under shaking with a speed of 200 rpm to 250 rpm for 16 hours to 18 hours or until the OD₆₀₀ value ranges from 2 to 6, and collecting the strain, for example, by centrifugation, to obtain the seed solution.

A sixth aspect of the present disclosure provides a method for producing alanyl-glutamine dipeptide, the method including:
catalyzing a reaction of glutamine and alanine methyl ester hydrochloride, in presence of the amino acid ester acyltransferase prepared by the method according to the fifth aspect of the present disclosure, to generate the alanyl-glutamine dipeptide.

In some specific embodiments, said catalyzing is carried out in a buffer system with a pH ranging from 8.0 to 9.0; and/or a temperature of said catalyzing ranges from 10 °C to 20°C.

In some preferred embodiments, a molar ratio of the glutamine to the alanine methyl ester hydrochloride is (1-5):1, and preferably 2:1; and/or the amino acid ester acyltransferase is a concentrated enzyme solution with a protein concentration of 5 mg/mL to 10 mg/mL, and a molar mass ratio of the glutamine to the concentrated enzyme solution is (1-5):1, and preferably 3:1.

Use of the promoter according to the first aspect the fusion gene according to the second aspect, or the recombinant expression vector according to the third aspect of the present disclosure in the preparation of *Pichia pastoris* engineered strain capable of secreting and expressing amino acid ester acyltransferase or in the production of alanyl-glutamine dipeptide.

On the basis of common knowledge in the art, the above-mentioned preferred conditions can be arbitrarily combined to obtain the preferred embodiments of the present disclosure.

The reagents and raw materials used in the present disclosure are commercially available.

The positive and progressive effect of the present disclosure lies in that, firstly, a novel inducible promoter with strong promoter activity is screened in this technical solution, and the enzymatic activity of the expressed amino acid ester acyltransferase was up to 670 U/mL. Secondly, a high-copy recombinant strain with a copy number of 50 was screened in the present technology. When the substrate was converted to form alanyl-glutamine dipeptide, the conversion rate of glutamine was up to 90%, the yield of alanyl-glutamine dipeptide was up to 50.5 mg/mL (which is twice as much as that in document 2 in the background art), and the yield of by-product tripeptide was 0.3%.

### Biological material deposit information

*Pichia pastoris* G6 of the present disclosure was deposited in the China Center for Type Culture Collection (CCTCC) on October 22, 2021. The deposit address is Wuhan University, Luojiashan, Wuchang, Wuhan City, Hubei Province, China, Postal Code 430072. The deposit number is CCTCC NO: M 20211308. The culture name is G6, and the classification name is *Pichiapastoris.*

### BRIEF DESCRIPTION OF DRAWINGS

FIG. 1 is a map of p9K, p9K-promoter, and p9K-promoter-AET vectors; panel A in FIG. 1 is a map of p9K, panel B in FIG. 1 is a map of p9K-promoter, and panel C in FIG. 1 is a map of p9K-promoter-AET.
FIG. 2 shows the content of alanyl-glutamine dipeptide in mg/mL converted from the ultrafiltered enzyme solution expressed by the fermentation of recombinant strains G6, G7, and K6 and G6 freeze-dried enzyme powder.
FIG. 3 shows the glutamine conversion rate and the yield of by-product tripeptides of the ultrafiltered enzyme solution expressed by the fermentation of recombinant strains G6, G7, and K6 and G6 freeze-dried enzyme powder
FIG. 4 shows the protein electrophoresis of the recombinant strains G6, G7, and K6 at different induction times.
FIG. 5 shows the protein electrophoresis of fermentation supernatant of recombinant strain G6 after induction for 96 h and membrane concentration.

### DESCRIPTION OF EMBODIMENTS

The present disclosure is further illustrated below by way of examples, but it is not limited to the scope of the described examples. The experimental methods in the following examples without specifying specific conditions are chosen according to conventional methods and conditions or according to product specifications.

**Table 1 Medium information:**

| Product Name | BMGY(1L) | BMMY(1L) |
|---|---|---|
| YNB | 13.4 g | 13.4 g |
| Biotin | 0.0004 g | 0.0004 g |
| Dipotassium hydrogen phosphate | 3g | 3g |
| Potassium dihydrogen phosphate | 11.8 g | 11.8 g |
| Methanol | - | 5 mL |
| Yeast powder | 10 | 10 |
| Peptone | 20 | 20 |
| Glycerin | 10 mL | - |

### Example 1: Transcriptome sequencing and acquisition of candidate promoter sequences

Strain culture: *Pichia pastoris* strain GS115 (purchased from Wuhan Miaoling Biotechnology Co. Ltd. Cat No.: T0073) was streaked on a YPD plate, and a single colony was picked and inoculated into BMGY expansion medium for overnight culture at 28°C and 250 rpm until the OD₆₀₀ was between 2 to 6, then transferred to BMMY for methanol induction culture for 96 h. The strain liquid of the BMGY expanded culture (control group) and the strain liquid of the BMMY induced culture (experimental group) were taken, respectively.

RNA extraction: the RNA of the control group and the experimental group were extracted with the bacterial RNA extraction kit of Qiagen. The extracted RNA samples were examined by 1% agarose gel and showed no protein contamination. The concentration of RNA was detected by Nanodrop (Thermo Fisher, Nanodrop 2000). The nucleic acid concentrations of the control group and experimental group were 213 ng/µl and 268 ng/µl, respectively, which met the requirements for sample presentation.

Transcriptome sequencing: the extracted RNA samples were entrusted to Beijing Novogene Bioinformatics Technology Co., Ltd. for transcriptome sequencing. The main steps were: the mRNA was purified by Sera-mag Magnetic Oligo (dT) Beads magnetic bead purification (Illumina), and the purified mRNA was fragmented by divalent cation treatment. Then the reverse transcriptase SuperScript II (Invitrogen), RNaseH (Illumina), DNA polymerase (Illumina), and random primers were added to synthesize double-stranded cDNA using the fragmented mRNA as a template, and the cDNA was purified using PCR purification kit. The synthesized cDNA was modified by end-filling and phosphorylation by adding T4 DNA polymerase, Klenow DNA polymerase, and T4 PNK, and the end-filled cDNA was purified. The terminally modified cDNA was endowed with an overhanging adenylate-A at the 3' end by the action of Klenow Exo (Illumina) to facilitate ligation with a linker, and the adenylated cDNA adapters were purified using MinElutePCR Purification Kit (Qiagen). The Illumina PE adapters were linked to the 3' end of the adenylated cDNA fragment using T4 DNA Ligase (Illumina), and the product was purified. The cDNA fragments with the adapters were separated by 2% agarose gel electrophoresis, and the cDNA fragments with a size of 200 bp ± 25 bp in the gel were purified and recovered. The cDNA fragments with adapters were enriched by PCR amplification. After the products were recovered and tested as qualified, they were used as a cDNA library for RNA-Seq sequencing. The cDNA library was sequenced using the paired-end strategy.

Sequencing data processing and analysis: a large number of sequence fragments (reads) could be obtained by RNA-seq. Firstly, the low-quality reads were removed, and the remaining high-quality reads were aligned with the published *Pichia pastoris* genome using sequence alignment software to find the reads matched with the unique site (uniquely mapped reads) on the genome, and the expression level of the gene was analyzed, and then the transcription level of each gene was normalized using the RPKM (Reads Per Kilobase per Million mapped reads) value.

Screening candidate genes: analysis of the differentially expressed genes in the experimental group and control group were performed, including GO enrichment analysis and KEGG enrichment analysis of the differential expressed genes. The transcriptome data and differentially expressed genes were analyzed to screen genes whose transcription levels were significantly up-regulated after methanol induction. Specifically, the genes with a significantly increased transcription level after methanol induction were obtained through the transcriptome data, and then the screened genes were aligned with the *Pichia pastoris* genome database and predicted using promoter software to find the promoter sequences located upstream of the genes. The main difficulty was to screen the candidate genes whose expression can be induced by methanol. Six genes whose transcript levels were significantly up-regulated (p<0.01) during the methanol induction period were finally screened out from a large number of genes. These six genes are distributed in different metabolic pathways (see Table 2 below).

**Table 2 Information of the promoter screened**

| Gene name | Chromosome | + strand/-strand | Initiation site | Termination site | Ratio (log2) |
|---|---|---|---|---|---|
| PpRNA RS07685 | chr3 | - | 1608584 | 1610707 | 3.51 |
| PpRNA. RS11281 | chr2-1 | - | 923474 | 924223 | 2.8 |
| PpRNA RS05760 | chr4 | + | 129794 | 130816 | 3.2 |
| PpRNA. RS04123 | chr4 | + | 301802 | 303793 | 6.7 |
| PpRNA. RS00891 | chr3 | - | 1980650 | 1981903 | 2.26 |
| PpRNA RS02207 | chr1 | - | 2417633 | 2418145 | 4.18 |

Acquisition of promoter region sequence: blast alignment was made between the six genes screened above and the *Pichia pastoris* genome, the promoter region was analyzed by promoter prediction software or NCBI gene annotation information, and the promoter sequences were downloaded. The numbering of the promoter sequences is shown in Table 3 below.

**Table 3 Promoter sequences and numbering**

| Name | Serial Number SEQ ID NO: |
|---|---|
| Promoter sequence of gene PpRNA RS07685 | 1 |
| Promoter sequence of gene PpRNA RS11281 | 2 |
| Promoter sequence of gene PpRNA RS05760 | 3 |
| Promoter sequence of gene PpRNA RS04123 | 4 |
| Promoter sequence of gene PpRNA RS00891 | 5 |
| Promoter sequence of gene PpRNA RS02207 | 6 |

### Example 2: Construction of AET expression vectors with different promoters and detection of AET enzyme activity

Primers were designed to amplify the six promoter sequences in Example 1 as well as the AET gene, and the primer sequences are shown in Table 4. The primers were synthesized by Wuhan Gene Create biology engineered Co., Ltd. The genome of *Pichia pastoris* GS115 was extracted with a yeast genome extraction kit (OMEGA), and the promoter sequence was amplified with PCR, the PCR system was: 18 µl of ddH₂O, 2.5 µl of forward primer, 2.5 µl of reverse primer, 2 µl of template, 2×Q5 mix (NEB) 25 µl, a total system of 50 µl. PCR program: 98°C for 1 min, 98°C for 15 s, 55°C for 30 s, 68°C for 1 min (30 cycles), 68°C for 7 min, 4°C ∞. The AET fragment was amplified by PCR using PUC57-AET (synthesized by GeneCreate Biology Science and Technology Co., Ltd.) plasmid as a template. The PCR system and PCR program were the same as above. The vector p9K uses pAOX1 to express the foreign proteins, which is currently the most commonly used strong promoter in *Pichia pastoris.* Firstly, the p9K plasmid was digested with BglII and BamHI to remove the original promoter pAOX1 and then ligated with the above-mentioned six promoter fragments. The ligation product was transformed into DH5α, and verified by colony PCR. The positive clones were picked and sent to GeneCreate Co., Ltd. for sequencing for further verification. The correctly sequencd vector was named p9k-promoter. P9k-promoter and p9K were digested with EcoRI and NotI and ligated with the AET fragment, the ligation product was transformed into DH5α, verified by colony PCR and further verified by sequencing. The Sequencing-verified plasmids were named p9k-promoter-AET and p9K-AET. The p9k-promoter-AET and p9K-AET were linearized with BglII restriction endonuclease and recovered by alcohol precipitation to concentrate the linearized vector to 500-1000 ng/µl. The plasmid was transformed into GS115 by electroporation. The transformation procedures were as follows: 10 µl of the linearized vector was added into 80 µl of GS115 electrotransformed competent cells, shocked at 1.5 kv for 5 ms, 1 ml of 1 M sorbitol was rapidly added, and the cells were recovered and cultured at 28-30°C for 2 h. The cells were coated on MD plates containing 100 µg/mL of G418 and cultured at 28-30°C for 2-3 days.

The transformants were inoculated into 25 mL of BMGY medium, cultured at 28-30°C and under shaking with a speed of 245 rpm for 16-18 h, and collected by centrifugation when the OD₆₀₀ was 2-6; 50 mL of BMMY medium (OD₆₀₀= 1.0) was added, cultured at 25-30°C under shaking with a speed of 245 rpm to start induction expression, methanol was supplemented every 24 h, and the final concentration of methanol was 0.5% to1%. The fermentation supernatant at 48 h and 96 h was taken to detect the enzyme activity of AET. The enzyme activity detection method was as follows: 9.9 ml of borate buffer solution with a final concentration of 100 mmol/L at pH 9.0 was put into a small beaker with a volume of 50 ml, magnetically stirred in a constant temperature magnetic water bath, and preheated at 25°C for 20 min. 100 mmol/L glutamine powder was added to dissolve completely; 0.1 ml of enzyme solution (the enzyme solution was diluted appropriately according to enzyme activity) was added, finally, 100 mmol/L of alanine methyl ester hydrochloride powder was added, and the mixture was reacted at 25°C for 5 min. After the reaction is completed, 1 ml of the reaction solution was taken into an EP tube and immediately added with an equal volume of 0.1 mM hydrochloric acid to terminate the reaction, and liquid phase detection was performed after water bath at 60°C for 10 min, producing 1 µmol of alanyl-glutamine dipeptide per minute as one unit of enzyme activity.

**Table 4 Primer sequences used to construct vectors**

| Primer Name | Primer Sequence (5'-3') | Serial Number SEQ ID NO: |
|---|---|---|
| PpRNA. RS07685Fp | aatgatatttgagggtgttagttact | 7 |
| PpRNA RS07685Rp | ttttgatgtttgatagtttgataagagtgaa | 8 |
| PpRNA. RS11281Fp | gatgagtatcaaaggggatttggtt | 9 |
| PpRNA RS11281Rp | gattaagagaaagtccaagtcgaag | 10 |
| PpRNA RS05760Fp | gtctttgtaaatagttatagttcagaaactgg | 11 |
| PpRNA. RS05760Rp | gatgatttattgaagtttccaaagttgaga | 12 |
| PpRNA RS04123Fp | gaattctttttttcagaccatatgaccg | 13 |
| PpRNA. RS04123Rp | ttttctcagttgatttgtttgtggg | 14 |
| PpRNA RS00891Fp | gaattagtgagataacagagttgggta | 15 |
| PpRNA. RS00891Rp | tgtgaatatcaagaattgtatgaacaagcaa | 16 |
| PpRNA. RS02207Fp | ttctggagtgtcaaaacagtagtgat | 17 |
| PpRNA RS02207Rp | cttagaatttttttttttgtcttggtggg | 18 |
| AETFp | ccggaattcatgactcgaagaggtggttccg | 19 |
| AETRp | aaatatgcggccgcaggctgaatcacaggaaggtca | 20 |

**Table 5 Enzymatic activity produced by AET expression vectors with different promoters**

| Expression vector | Amount of alanyl-glutamine dipeptide product (mg/mL) | Enzyme activity (U/mL) |
|---|---|---|
| p9K-RS07685-AET | 2.46 | 454 |
| p9K-RS11281-AET | 1.28 | 236 |
| p9K-RS05760-AET | 0.99 | 182 |
| p9K-RS04123-AET | 1.68 | 310 |
| p9K-RS00891-AET | 1.50 | 277 |
| p9K-RS02207-AET | 3.64 | 670 |

As can be seen from Table 5, the amount of alanyl-glutamine dipeptide product and the enzyme activity obtained by promoter RS02207 were higher than those of other promoters.

Comparative Example 1: Comparison of the amount of alanyl-glutamine dipeptide product and enzyme activity after expression of P9K-AET and p9K-RS02207-AET in GS115

**Table 6. Amount of alanyl-glutamine dipeptide product and enzyme activity of different promoters in Comparative Example 1.**

| Expression vector | Amount of alanyl-glutamine dipeptide product (mg/mL) | Enzyme activity (U/mL) |
|---|---|---|
| p9K-AET | 1.92 | 354 |
| p9K-RS02207-AET | 3.64 | 670 |

As can be seen from Table 6, the promoter RS02207 was more active than the AOX1 promoter of p9K, and the AET enzyme activity expressed by the former was about 1.9 times that of the latter.

Wherein, the nucleotide sequence of promoter RS02207(SEQ ID NO: 6) is as follows:

ttctggagtg tcaaaacagt agtgataaaa ggctatgaag gaggttgtct aggggctcgt 60

ggaggaaagt gattcaaaca gacctgccaa aaagagaaaa aagagggaat ccctgttctt 120

tccaatggaa atgacgtaac tttgacttga aaaatacccc aaccagaagg gttcaaactc 180

aacaaggatt gcgtaattcc tacaagtagc ttagagctgg gggagagaca actgaaggca 240

gcttaacgat aactcggggg gattggtgca cgactcgaaa ggaggtttct tagtcttgta 300

acctcttttt tccagaggct attcaagatt cataggcgat atcgatgtgg agaagggtga 360

acaatataaa aggctggaga gatgtcaatg aagcggctgg atagatttca aattttctag 420

atttcagagt aatcgcacaa aacgaaggaa tcacaccaag acaaaaaaaa aaattctaag 480.

### Example 3: Expression of p9K-RS02207-AET in Pichiapastoris X33

The constructed p9K-RS02207-AET and p9K-AET were linearized with salI and electroporated into the X33 host. X33 is a wild-type *Pichia pastoris* host. The electrotransformation method is as described in Example 2. After electroporation, the cells were plated on YPD plates, and positive transformants were screened with 100 µg/mL G418 antibiotic. The X33 transformants of p9K-RS02207-AET and the X33 transformants of p9K-AET were picked into BMGY seed culture medium, respectively, cultured at 25-30°C under shaking with a speed of 245 rpm until OD₆₀₀ was 2 to 6, the strains were collected by centrifugation, added into BMMY medium for induction expression, supplemented with methanol every 24 h, and the final concentration of methanol was 0.5%-1%. After 96 h of induction, the fermentation supernatant was collected and the enzymatic activity of the α-amino acid ester acyltransferase was detected according to the enzymatic activity detection method described in Example 2.

Comparative Example 2: Comparison of the amount of alanyl-glutamine dipeptide product and enzyme activity after expression of P9K-AET and p9K-RS02207-AET in X33

**Table 7. Amount of alanyl-glutamine dipeptide product and enzyme activity of different promoters in Comparative Example 2**

| Expression vector | Amount of alanyl-glutamine dipeptide product (mg/mL) | Enzyme activity (U/mL) |
|---|---|---|
| p9K-AET | 1.65 | 303 |
| p9K-RS02207-AET | 3.10 | 570 |

It can be seen from Table 7 that the amino acid ester acyltransferase expressed by the promoter RS02207 was higher than that expressed by the AOX1 promoter, and the yield of the alanyl-glutamine dipeptide produced was higher.

### Example 4: Expression of p9K-RS02207-AET in Pichiapastoris KM71

The constructed p9K-RS02207-AET and p9K-AET were linearized with salI and then electroporated into KM71 host. KM71 is a MutS-type histidine-deficient *Pichia pastoris* host. The electroporation method is as described in Example 2. After electroporation, the cells were plated on MD culture plates, and positive transformants were selected using the histidine-free medium. The KM71 transformants of p9K-RS02207-AET and KM71 transformants of p9K-AET were respectively picked into BMGY seed medium, espectively, and cultured at 25-30°C under shaking with a speed of 245rpm until the OD₆₀₀ was 2 to 6. The strains were collected by centrifugation and added into BMMY medium for induction express, supplemented with methanol every 24 h, and the final concentration of methanol was 0.5%-1%. After 96 h of induction, the fermentation supernatant was collected and the enzymatic activity of the α-amino acid ester acyltransferase was detected according to the enzymatic activity detection method described in Example 2.

Comparative Example 3: Comparison of the amount of alanyl-glutamine dipeptide product and enzyme activity after expression of P9K-AET and p9K-RS02207-AET in KM71

**Table 8. Amount of alanyl-glutamine dipeptide product and enzyme activity of different promoters in Comparative Example 3**

| Expression vector | Amount of alanyl-glutamine dipeptide product (mg/mL) | Enzyme activity (U/mL) |
|---|---|---|
| p9K-AET | 2.11 | 388 |
| p9K-RS02207-AET | 3.40 | 626 |

It can be seen from Table 8 that the amino acid ester acyltransferase expressed by the promoter RS02207 was higher than that expressed by AOX1 promoter, and the yield of the alanyl-glutamine dipeptide produced was higher.

### Example 5: Multi-copy recombinant strain screening

The constructed p9K-RS02207-AET vector was linearized with salI and electroporated into GS115 and KM71. Multi-copy inserts were screened by increasing the G418 concentration. The electroporated transformants were transferred to YPD plates with a G418 concentration gradient of 1.5 mg/mL, 2 mg/mL, and 4 mg/mL, respectively, and cultured at 28°C for 2-3 days to observe the growth of the colonies. Colonies that could grow normally on the YPD plate with 4 mg/mL G418 accounted for 1%-10%.

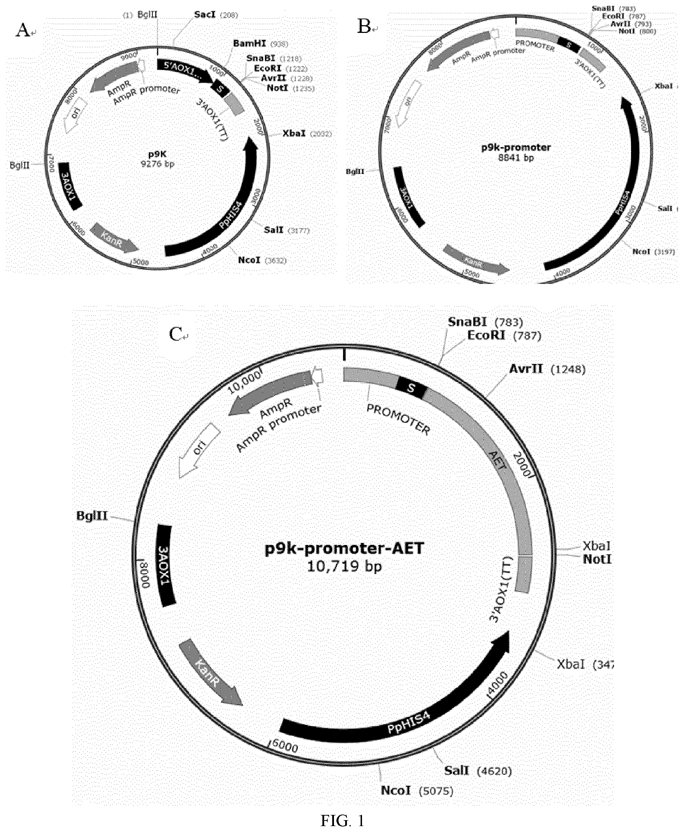

The glyceraldehyde-3-phosphate dehydrogenase gene GAP was used as an internal reference gene, and the cloning plasmids pMD 19T-GAP and pMD 19T-AET of GAP and AET were constructed, respectively. Specifically, the yeast genome of the host GS115 or KM71 was extracted and the GAP gene was amplified using specific primers (see Table 9) and the primer sequences were GAPRT-Fp and GAPRT-Rp. Using the pPIC9K-AET plasmid as a template, the AET gene was amplified using specific primers, and the primer sequences were AETRT-Fp and AETRT-Rp (see Table 9). The GAP fragment and the AET fragment were ligated to pMD 19T, respectively, then transformed into DH5α. The next day, the transformants were picked and verified successfully to obtain the standard plasmid strain. The vector map was shown in FIG. 1, panel A in FIG. 1 was a map of p9K, panel B in FIG. 1 was a map of p9K-promoter, and panel C in FIG. 1 was a map of p9K-promoter-AET.

Standard plasmids pMD 19T-GAP and pMD 19T-AET were extracted, and the concentrations of the extracted plasmids containing GAP and AET genes were determined using a micro-nucleic acid quantitative instrument, and the copy number of plasmid was calculated according to the copy number conversion formula: copy number = 6.023 × 10¹⁴× plasmid mass concentration/(660×M), where M refers to the length of gene (bp), plasmid mass concentration is in (ng/µl). The plasmids were diluted to 10³, 10⁴, 10⁵, 10⁶, 10⁷, and 10⁸ copies per µL. Fluorescence quantitative PCR detection was performed using 1 µL of plasmid solution of each concentration as a template and GAPRT-Fp and GAPRT-Rp, AETRT-Fp, and AETRT-Rp as primers. Reaction system: 2×master mix 10 µl, 1 µl each of forward primer and reverse primer, 5 µl of DNA template, and double distilled water was added to a total volume of 20 µl. Reaction conditions: pre-denaturation at 95°C for 2 min, denaturation at 95°C for 15 s, annealing at 60°C for 15 s, extension at 68°C for 30 s, for a total of 40 cycles, melting curve conditions: 95°C for 10 s, 65°C for 1 min, 97°C for 1 s, with an increase of 5°C per cycle. A standard curve was drawn with the logarithm of the copy number as the abscissa and the Ct value as the ordinate.

The multi-copy recombinant strain genome were extracted and subjected to fluorescent quantitative PCR detection in triplicate for each sample. The obtained ct values were substituted into two standard curves respectively, and the starting template copy number of the GAP gene and AET gene in the sample were calculated. Since the GAP gene existed in *Pichia pastoris* in a single copy, the copy number of the AET gene in the *Pichia pastoris* genome = the starting template copy number of the AET gene/the starting template copy number of the GAP gene.

Finally, three AET high-copy recombinant strains were screened, namely G6, G7, and K6, the host of G6 and G7 was *Pichia pastoris* GS1 15, the host of K6 was *Pichia pastoris* KM71, and the copy numbers of AET gene were 50, 19 and 17, respectively (see Table 10).

**Table 9: Primer for fluorescent quantitative detection of gene copy number**

| Primer Name | Primer sequence | Serial Number SEQ ID NO: |
|---|---|---|
| GAPRT-Fp | ggtattaacggtttcggacgtattg | 21 |
| GAPRT-Rp | gatgttgacagggtctctctcttgg | 22 |
| AETRT-Fp | ggtatccatgctgcctggtttac | 23 |
| AETRT-Rp | gcgatctgatggacgtcgtagtt | 24 |

**Table 10: Copy number of high-copy amino acid ester acyltransferase recombinant expression strain**

| Recombinant strain | starting template copy number of GAP gene | starting template copy number of AET gene | copy number of AET gene |
|---|---|---|---|
| G6 | 88243164.78 | 4453522222.60 | 50 |
| G7 | 101608786.98 | 1920731099.41 | 19 |
| K6 | 264346.27 | 4436162.92 | 17 |

### Example 6: Inducing expression of recombinant strain to produce enzyme

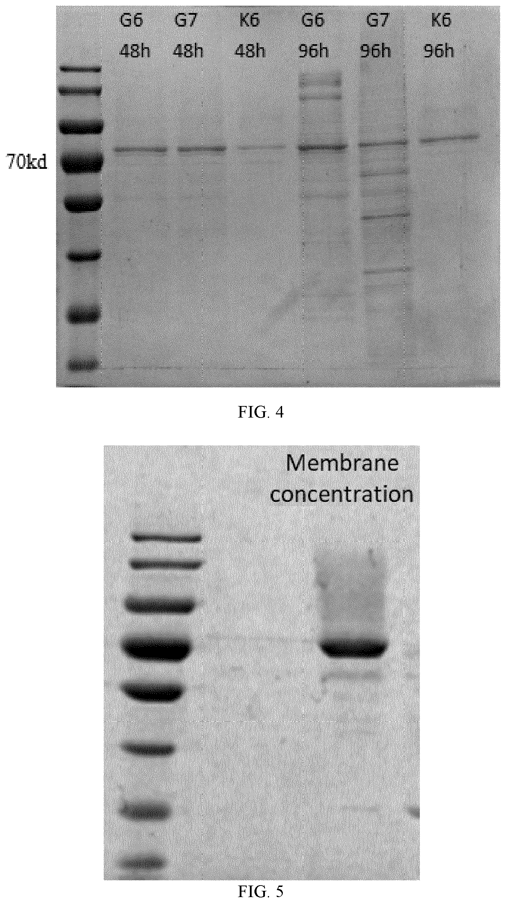

A single colony of recombinant strain G6 was inoculated into 25 mL of BMGY medium, and cultured at 25-30°C for 16-18 h under shaking with a speed of 245 rpm. The strains were collected by centrifugation when the OD₆₀₀ was 2 to 6. 50 mL BMMY medium (OD₆₀₀=1.0) was added, cultured at 25-30°C under shaking with a speed of 245 rpm to start inducing the expression of target protein, methanol was supplemented every 24 h, and the final concentration of methanol was 0.5% to 1%. 1 mL of samples were taken at 48 h and 96 h respectively to detect the strain concentration and protein concentration, and some samples were subjected to SDS-PAGE electrophoresis. The results were as shown in FIG. 4. Under the above conditions, the OD₆₀₀ of the fermentation broth was about 20-30, the protein concentration was 2.0 mg/mL-2.5 mg/mL after 96 h of induction. After ultrafiltration concentration, the protein concentration was 5.5 mg/mL-7 mg/mL. As can be seen from the SDS-PAGE electrophoresis diagram (FIG. 5), the amino acid ester acyltransferase was successfully secreted and expressed, with a band of interest at a theoretical size of 71 kd.

### Example 7: Conversion of alanyl-glutamine dipeptide from recombinant strain fermentation broth

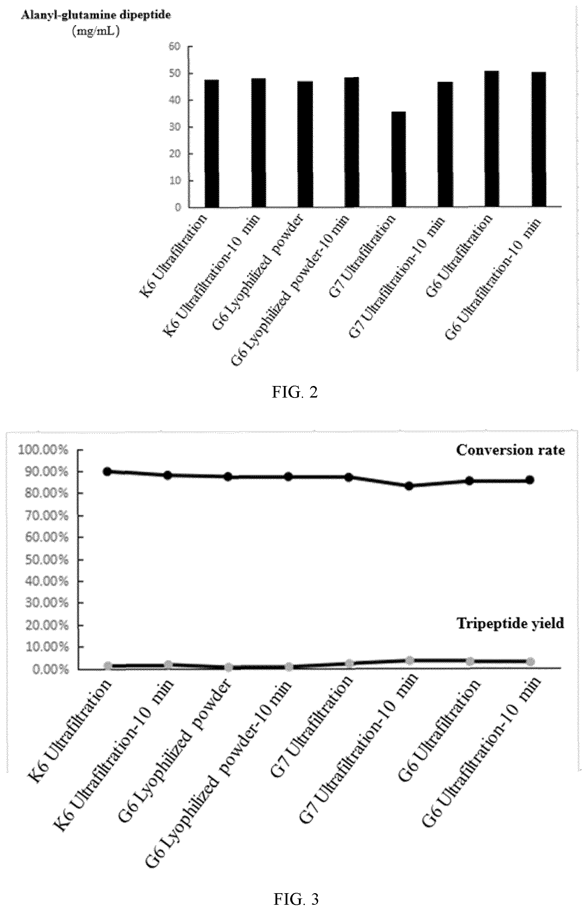

30 mM glutamine was dissolved with 61.6 mL water, pH was adjusted to 8.0-9.0 with ammonia water, the temperature was controlled to 10°C-20°C, 2 mL ultrafiltered enzyme solution was added, alanine methyl ester hydrochloride solution (36 mM, 25 mL in total) was added using a peristaltic pump at low speed, and pH and temperature were controlled in the process. After the dropwise addition was completed, 1 mL of the reaction solution was taken, 1 mL of methanol was added, and the mixture was placed in a water bath at 60°C for 10 min to terminate the reaction. 10 min after the completion of the dropwise addition, 1 mL of the reaction solution was taken, 1 mL of methanol was added, and the mixture was placed in a water bath at 60°C for 10 min to terminate the reaction. The contents of alanyl-glutamine dipeptide, glutamine, and tripeptide in the transformed samples were detected by HPLC. Results were as shown in FIG. 2 and FIG. 3, the supernatant induced and expressed by the selected three recombinant strains could effectively convert glutamine and alanine methyl ester hydrochloride into alanyl-glutamine dipeptide, the highest conversion rate of glutamine could reach 90%, the yield of alanyl-glutamine dipeptide could reach 50.5 mg/mL, and the yield of by-product tripeptide was 0.3%.

Although specific embodiments of the present disclosure have been described above, it will be understood by those skilled in the art that these are merely examples and that various changes or modifications may be made to these embodiments without departing from the principles and spirit of the present disclosure. Therefore, the protection scope of the present disclosure is defined by the appended claims.

## Claims

1. A promoter, wherein a nucleotide sequence encoding the promoter is as shown in SEQ ID NO: 6.

2. A fusion gene, comprising:
a promoter, and an amino acid ester acyltransferase,
wherein the nucleotide sequence of the promoter is as shown in SEQ ID NO: 6, and wherein the nucleotide sequence of the amino acid ester acyltransferase is as shown in SEQ ID NO: 25.

3. A recombinant vector, comprising the promoter according to claim 1 or the fusion gene according to claim 2.

4. The recombinant vector according to claim 3, wherein a backbone plasmid of the recombinant vector is pGAPZαA, pPICZαA, pPIC9, or p9K;
preferably, the backbone plasmid is p9K.

5. A *Pichia pastoris* engineered strain, comprising the recombinant expression vector according to claim 3 or 4,
wherein an expression host of the *Pichia pastoris* engineered strain is a wild-type host, a histidine-deficient host, or a protease-deficient host.

6. The *Pichia pastoris* engineered strain according to claim 5, wherein:
the wild-type host is X33;
the histidine-deficient host is KM71 or GS115;
the protease-deficient host is SMD1163, SMD1165, or SMD1168;
preferably, the expression host of the *Pichia pastoris* engineered strain is *Pichia pastoris* strain GS115.

7. The *Pichia pastoris* engineered strain according to claim 5 or 6, wherein the *Pichia pastoris* engineered strain is *Pichia pastoris* engineered strain G6 deposited with a deposit number of CCTCC NO: M 20211308.

8. A method for preparing an amino acid ester acyltransferase, comprising:
culturing a seed solution comprising the *Pichia pastoris* engineered strain according to any one of claims 5 to 7 in a BMMY medium, and
adding, when the OD₆₀₀ value is 0.5 to 2, such as 1.0, an inducer such as methanol to induce expression of the amino acid ester acyltransferase; wherein a final concentration of the inducer ranges preferably from 0.5% to 1%.

9. The method according to claim 8, wherein:
a duration of said induction expression ranges from 48 hours to 120 hours, such as 96 hours, or said induction expression lasts until the OD₆₀₀ value ranges from 20 to 30, to obtain a fermentation broth containing the amino acid ester acyltransferase;
preferably, methanol is supplemented every 12 hours to 24 hours.

10. The method according to claim 8 or 9, further comprising:
concentrating the fermentation broth to obtain a concentrated enzyme solution,
preferably, said concentrating is ultrafiltration concentrating.

11. The method according to any one of claims 8 to 10, wherein the seed solution is prepared by:
(a) inoculating the *Pichia pastoris* engineered strain into a medium, such as BMGY medium;
(b) culturing at 25 to 30°C under shaking with a speed of 200 rpm to 250 rpm for 16 hours to 18 h hours or until the OD₆₀₀ value ranges from 2 to 6, and collecting the strain, for example, by centrifugation, to obtain the seed solution.

12. A method for producing alanyl-glutamine dipeptide, the method comprising:
catalyzing a reaction of glutamine and alanine methyl ester hydrochloride, in presence of the amino acid ester acyltransferase prepared by the method according to claims 5 to 7, to generate the alanyl-glutamine dipeptide.

13. The method according to claim 12, wherein said catalyzing is carried out in a buffer system with a pH ranging from 8.0 to 9.0; and/or a temperature of said catalyzing ranges from 10°C to 20°C.

14. The method according to claim 12 or 13, wherein a molar ratio of the glutamine to the alanine methyl ester hydrochloride is (1-5): 1, and preferably 2: 1; and/or
the amino acid ester acyltransferase is a concentrated enzyme solution with a protein concentration of 5 mg/mL to 10 mg/mL, and a molar mass ratio of the glutamine to the concentrated enzyme solution is (1-5): 1, and preferably 3:1.

15. Use of the promoter according to claim 1, the fusion gene according to claim 2, or the recombinant expression vector according to claim 3 or 4 in the preparation of *Pichia pastoris* engineered strain capable of secreting and expressing amino acid ester acyltransferase or in the production of alanyl-glutamine dipeptide.
